(19) Europäisches Patentamt
European Patent Office
Office européen des brevets

(11) Publication number: **0 082 667**
**A1**

(12) # EUROPEAN PATENT APPLICATION

(21) Application number: 82306685.7

(22) Date of filing: 14.12.82

(51) Int. Cl.³: **A 61 K 31/70**
//(A61K31/70, 31/19)

(30) Priority: 18.12.81 GB 8138295

(43) Date of publication of application: 29.06.83 Bulletin 83/26

(84) Designated Contracting States: CH DE FR GB IT LI NL

(71) Applicant: BEECHAM GROUP PLC
Beecham House Great West Road
Brentford Middlesex(GB)

(72) Inventor: Harnden, Michael Raymond
47 Guildford Road
Horsham Sussex(GB)

(72) Inventor: Bailey, Stuart
14 Overdale
Dorking Surrey(GB)

(74) Representative: Russell, Brian John et al,
European Patent Attorney Beecham Pharmaceuticals
Great Burgh Yew Tree Bottom Road
Epsom Surrey, KT18 5XQ(GB)

(54) **Pharmaceutical compositions.**

(57) A pharmaceutical composition comprising a compound of formula (II):

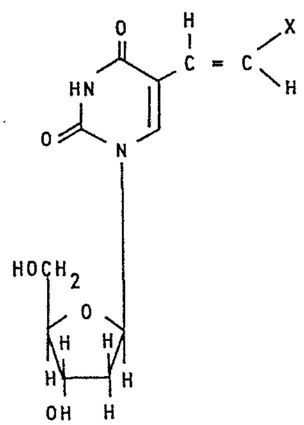

(II)

or a pharmaceutically acceptable ester thereof, wherein X is Cl, Br, I or H,
and probenecid or a pharmaceutically acceptable salt thereof,
together with a pharmaceutically acceptable carrier or excipient; is useful in treating viral infections.

EP 0 082 667 A1

# PHARMACEUTICAL COMPOSITIONS

This invention relates to a pharmaceutical composition having antiviral activity.

U.K. Patent Specification No. 1,601,020 discloses a small group of nucleoside analogues, namely 5-(2-halogenovinyl)-2'-deoxyuridines, which have antiviral activity selective against herpes viruses. One of these compounds, *E*-5-(2-bromovinyl)-2'-deoxyuridine (BVDU), has particularly good activity, but suffers from two disadvantages. Firstly, it is rapidly excreted in animal urine and, secondly, we have discovered that it is transformed *in vivo* to a metabolite with little or no antiviral activity.

We have now found that, surprisingly, both the above disadvantages can be minimised by administering the deoxyuridine compound to an animal in combination with probenecid or a pharmaceutically acceptable salt thereof.

Probenecid, of formula (I):,

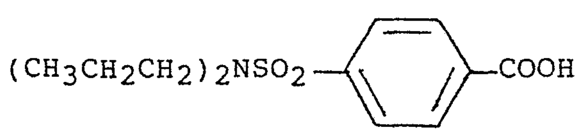

(I)

is known to block the renal tubular secretion of penicillin, but there is no evidence in the prior art that it could inhibit the transformation *in vivo* of nucleosides or nucleoside analogues to metabolites thereof.

Accordingly, the present invention provides a pharmaceutical composition comprising a compound of formula (II):

(II)

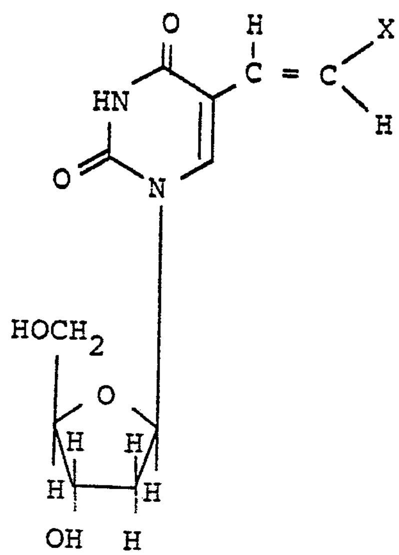

or a pharmaceutically acceptable ester thereof, wherein X is Cl, Br, I or H, and probenecid or a pharmaceutically acceptable salt thereof, together with a pharmaceutically acceptable carrier or excipient.

Preferably, X is Br.

Suitable pharmaceuticall acceptable salts of probenecid include alkali or alkaline earth metal salts, such as sodium or calcium, or ammonium or substituted ammonium salts.

Probenecid inhibits the transformation of the compound of formula (II) to its inactive metabolite, especially when both compounds are administered by the oral route. This results in higher concentrations of the compound of formula (II) in animal tissues and, therefore, improved antiviral activity.

The weight ratios of the compound of formula (II) or ester thereof to probenecid or a salt thereof in the composition of the invention may vary widely. A suitable ratio range is from 2:1 to 1:10 and a preferred range is from 1:1 to 1:3.

The composition of the invention is preferably given by the oral route, and it may be compounded in the form of syrups, tablets and capsules. When the composition is in the form of a tablet, any pharmaceutical carrier suitable for formulating such solid compositions may be used, for example magnesium stearate, starch, lactose, glucose, rice, flour and chalk. The composition may also be in the form of an ingestible capsule, for example of gelatin, to contain the compound, or in the form of a syrup, a solution or a suspension. Suitable liquid pharmaceutical carriers include ethyl alcohol, glycerine, saline and water to which flavouring or colouring agents may be added to form syrups.

The composition of the invention may also be presented with a sterile liquid carrier for injection.

Preferably, the compositions of this invention are in unit dosage form or in some other form that the patient may administer to himself a single dose. A suitable dosage unit might contain from 50 mg to 1 g of each active ingredient, for example 100 to 500 mg. Such doses may be administered 1 to 4 times a day or more usually 2 or 3 times a day.

Accordingly, in a further aspect of the invention, there is provided a method of treating viral infections in human or non-human animals which comprises administering to the animal an effective amount of a compound of formula (II) or a pharmaceutically acceptable ester thereof, in combination with an effective amount of probenecid or a pharmaceutically acceptable salt thereof.

The following Examples illustrate the invention.

Example 1

(A) Experimental Procedure

A single 100 mg/kg dose of BVDU, alone or in combination with 100 mg/kg or 200 mg/kg of probenecid, was administered as a suspension in carboxymethyl cellulose to groups of 3 starved female Balb/c mice. Blood was collected at 30, 75 (or 85) and 120 minutes after dosing and the serum separated and stored at -20°C.

(B) Biological Assay

Heat inactivated sera from single mice were titrated in plaque inhibition assays using the KOS strain of Herpes simplex type 1 virus in Vero cell monolayers. Antiviral activity is calculated by extrapolation from a standard inhibition dose response curve for BVDU and is quoted as the equivalent concentration of BVDU (in µg/mL) giving the same activity in cell culture.

HPLC Assay

Instrument: Waters H.P.L.C.
Column: RCM 100 $C_{18}$ Reverse Phase Radical Compression Column
Solvents: Solvent (A) 0.05M Ammonium Acetate at pH4.5
Solvent (B) 80% Methanol, 10% Water, 10%(A)
Conditions: 50% (B)
Detector wavelength: 250 mµ
Solvent Flow: 1 and 3 mL/min.

Mouse serum samples (90 µL each) were treated with 16% aqueous trichloroacetic acid solution (90 µL) to deposit all proteins and the samples spun down using a micro centrifuge. The supernatant portions were assayed by injection (20 µL) into a reverse phase HPLC column). The amounts of Probenecid were determined using a solvent flow of 3 mL/min, when Probenecid had a retention time of 11.5 min. This flow rate however, showed no separation of BVDU from its metabolite and the solvent flow rate was decreased to 1 mL/min. Under these conditions, the BVDU metabolite had retention time of 10.8 min and BVDU 11.6 min. The amounts of BVDU and Probenecid were calculated by comparison with an injected known amount of each drug. The amounts of BVDU metabolite were calculated by assuming the same detector response as BVDU. All calculations were then extrapolated to give the amount of each component present in each serum sample in µg/mL.

Experiment 1

Mice dosed with BVDU and serum assayed at 30 and 85 minutes after drug administration.

| Time after dosing (mins) | Antiviral activity in serum (BVDU equiv (µg/mL) ± Standard Deviation) | HPLC Determinations | |
|---|---|---|---|
| | | BVDU Conc (µg/mL) in serum | Metabolite conc (µg/mL) in serum |
| 30 | 39.8 ± 10.6 | 38.0 | 26 |
| 85 | 4.7 ± 1.4 | 3.9 | 16 |

Thus the antiviral activity in the serum can be accounted for entirely by the BVDU present (as determined by HPLC) and the metabolite appears to possess no significant antiviral activity.

Experiment 2

Mice dosed with BVDU or combinations of BVDU and Probenecid and the serum assayed by HPLC at 30, 75 and 120 minutes after drug administration.

| Material Administered (dose in (mg/kg) | Time after dosing (mins) | Concentration (µg/mL) in Serum | | |
|---|---|---|---|---|
| | | BVDU | BVDU Metabolite | Probenecid |
| BVDU (100) | 30 | 26.1 | 16.5 | - |
| BVDU (100) + Probenecid (100) | 30 | 20.8 | 11.9 | 5.4 |
| BVDU (100) + Probenecid (200) | 30 | 19.3 | 12.3 | 5.7 |
| Probenecid (200) | 30 | - | - | 3.8 |
| BVDU (100) | 75 | 13.0 | 20.7 | - |
| BVDU (100) + Probenecid (100) | 75 | 15.1 | 23.9 | 2.3 |
| BVDU (100) + Probenecid (200) | 75 | 14.3 | 15.0 | 3.3 |
| Probenecid (200) | 75 | - | - | 3.2 |
| BVDU (100) | 120 | 3.6 | 25.1 | - |
| BVDU (100) + Probenecid (100) | 120 | 8.5 | 28.5 | 1.5 |
| BVDU (100) + Probenecid (200) | 120 | 11.4 | 16.6 | 2.1 |
| Probenecid (200) | 120 | - | - | 2.3 |
| Undosed Control | - | - | - | - |

## Conclusions

Although at 120 minutes after dosing higher concentrations of BVDU are present in the sera of animals treated with either combination of BVDU and probenecid than in the sera of those treated with BVDU alone, the concentrations of the biologically inactive metabolite are lower in the sera of animals treated with the 100 mg/kg BVDU : 200 mg/kg probenecid combination at 75 and 120 minutes after dosing.

Since only BVDU and none of the metabolite was detected in the urine of the animals, the lower concentrations of the metabolite in the sera of animals treated with the 100 mg/kg BVDU : 200 mg/kg probenecid combination cannot be attributable to increased renal excretion and must be due to inhibition of the formation of the metabolite from BVDU.

Example 2

Activity of BVDU/Probenecid in Virus-infected Animals

1. Mouse Ear Herpesvirus Model (Reference, H.J. Field, Antimicrob. Ag. Chemother., 21, 744 (1982))

This model is based upon comparisons of viral replication in, and inflammation of, the ears of untreated or treated mice after infection with herpesvirus.

Procedure

The left pinna of female Balb/c mice were infected intradermally with $10^5$ PFU of herpes simplex virus type 1 (strain SC-16) suspended in 20µl of Eagle minimum essential medium. The mice were treated orally 3 times daily from the time of infection until day +4 with BVDU at 100mg/kg/dose or a 1:2 mixture of BVDU and probenecid (100/200mg/kg/dose). At selected times after infection groups of three mice were killed and the ear thickness of both the left and right ears measured using a micrometer gauge. The left ears were then removed and placed individually into 3ml of phosphate buffered saline. Samples were homogenised and sonicated. After centrifugation the supernatant was removed and assayed immediately in a plaque assay in Vero cells.

## Results

In two experiments, Experiments A and B, a lower virus titre was found from day +4 onwards in the ears of infected mice that had been treated with the BVDU/probenecid mixture than in the ears of those that had been administered the same dose of BVDU alone (Table A).

Additionally, the BVDU/probenecid mixture caused a marked reduction in ear thickness, whereas BVDU alone had no effect. With the mixture a statistically significant reduction in inflammation was observed on days 7, 8 and 9 (p 0.01) and days 11 and 12 (p .0.05) post infection (Table B).

## Example 3

### Hairless Mouse Cutaneous Herpesvirus Model

When hairless mice are infected with herpesvirus by dermal scarification, vesicles appear at the site of inoculation 6 days later. These rapidly coalesce to form a spreading unilateral lesion involving one hind quarter, leading eventually to ascending paralysis and death. The effect of treatments is assessed by a scoring method as lesions develop.

### Procedure

Groups of 10 adult hairless (HrHr) female mice were anaesthetized using ether and then infected with herpes simplex virus type 1 (strain SC-16) by scarifying an area $\approx 1cm^2$ at a site parallel with the top of the left hind limb, just below the midline, and introducing a drop of virus-containing solution to this site. Single daily doses of placebo, BVDU (100mg/kg/dose) or a 1:2 mixture of BVDU and probenecid (100/200mg/kg/dose) were administered orally for seven days, commencing 24 hours after infection. From 4 days after infection individual mice were examined daily and scored according to the severity of infection.

### Results

A slower progression of the cutaneous infection was observed in those mice treated with the BVDU/probenecid mixture than in those treated with BVDU alone (Table C).

TABLE A Effect of oral treatment with BVDU and a 1:2 BVDU/Probenecid mixture on virus replication in the mouse ear following inoculation of the pinna with HSV-1 (SC16)

| | Time post inoculation days | Virus titre ($\log_{10}$ p.f.u./ear)[a] | | |
|---|---|---|---|---|
| | | CMC[b] | BVDU[b] (100mg/kg/dose) | BVDU/Probenecid[b] (100/200mg/kg/dose) |
| Experiment A | 1 | 3.55 | 3.5 | 2.55 |
| | 2 | 4.2 | 3.0 | 3.0 |
| | 3 | 3.9 | 2.9 | 3.1 |
| | 4 | 3.7 | 3.3 | 2.5 |
| | 7 | 1.3 | 1.3 | 0.3 |
| Experiment B | 1 | 3.0 | 4.1 | 2.9 |
| | 2 | 4.1 | 3.0 | 3.2 |
| | 3 | 4.05 | 3.3 | 2.9 |
| | 4 | 3.8 | 3.9 | 2.8 |
| | 7 | 0 | 0 | 0 |

a Each value represents the mean virus titre from 5 animals

b Administered orally 3 x daily from day 0→ day 4.

TABLE B  Effect of oral treatment with BVDU and a 1:2 BVDU/Probenecid mixture on ear inflammation following inoculation of the pinna with HSV-1 (SC16)

| Time post inoculation (days) | Difference in ear thickness between uninfected and infected ears (mm x $10^{-2}$)[a] | | |
|---|---|---|---|
| | CMC[b] | BVDU[b] (100mg/kg/dose) | BVDU/Probenecid[b] (100/200mg/kg/dose) |
| 1 | 10.6 | 10.1 | 8.9 |
| 2 | 12.1 | 14.2 | 10.3 |
| 3 | 11.4 | 13.4 | 8.8 |
| 4 | 11.3 | 15.1 | 9.8 |
| 7 | 19.7 | 20.7 | 14.5 |
| 8 | 20.7 | 20.8 | 16.1 |
| 9 | 19.4 | 20.2 | 14.3 |
| 11 | 18.6 | 16.1 | 11.1 |
| 12 | 14.7 | 14.0 | 9.5 |
| 14 | 10.3 | 10.1 | 7.7 |

a Each value represents the mean result obtained from 8 to 10 mice

b Administered orally 3 x daily from day 0 → day 4.

TABLE C Effect of Single Daily Doses of BVDU and a BVDU/Probenecid Mixture given Orally for Seven Days to Hairless Mice Infected Cutaneously with HSV-1 (SC16)

Day +4

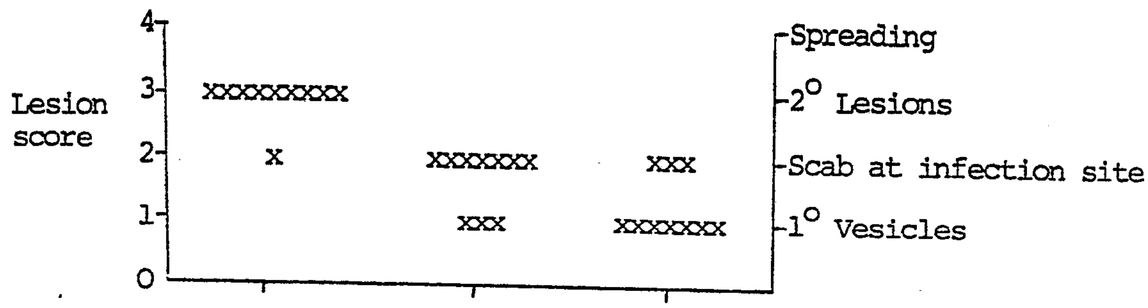


Day +8

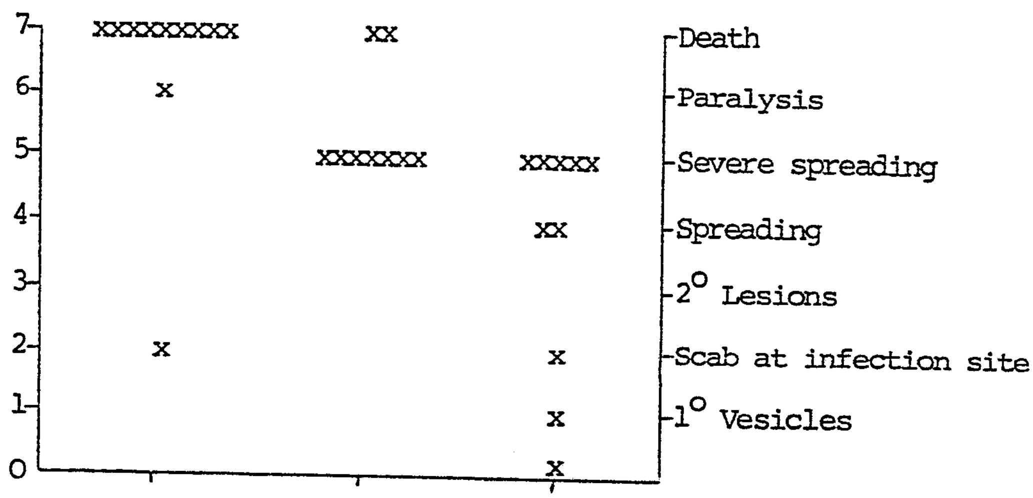


Day +11

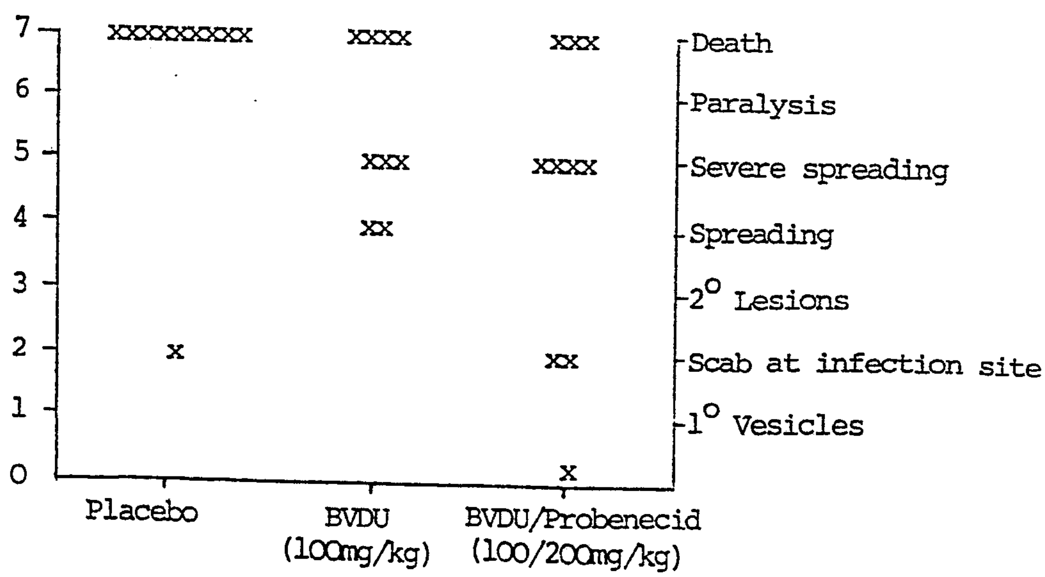

Claims



1. A pharmaceutical composition comprising a compound of formula (II):

(II)

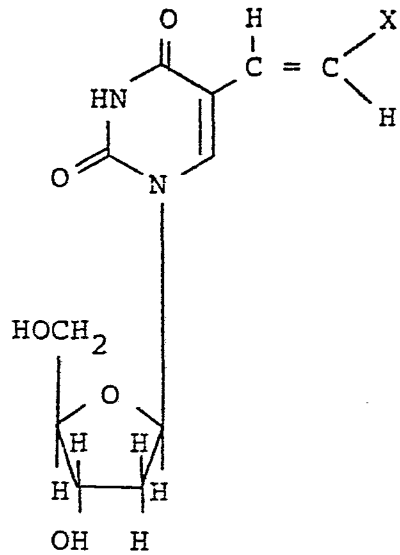

or a pharmaceutically acceptable ester thereof, wherein X is Cl, Br, I or H, and probenecid or a pharmaceutically acceptable salt thereof, together with a pharmaceutically acceptable carrier or excipient.

2. A composition according to claim 1, in which X is Br.

3. A composition according to claim 1 or claim 2, in which the weight ratio of compound of formula (II) or ester thereof to probenecid or salt thereof is from 2:1 to 1:10.

4. A composition according to any one of claims 1 to 3 in unit dosage form.

5. A composition according to any one of claims 1 to 4 for use in the treatment of viral infections.

European Patent Office

# EUROPEAN SEARCH REPORT

0082667
Application number

EP 82 30 6685

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | "Modern drug encyclopedia and therapeutic index", 8th Edition, 1961, The Reuben H. Donnelley Corporation, page 1075, New York (USA); *Page 1075: "Remanden 100"; "Remanden 250"* | 1-5 | A 61 K 31/70 // (A 61 K 31/70 A 61 K 31/19 ) |
| | --- | | |
| A | UNLISTED DRUGS, vol. 23, no. 8, August 1971, page 119c, Chatham, New Jersey (USA); *Page 119c: "Tofaben* | 1-5 | |
| | --- | | |
| D,A | GB-A-1 601 020 (THE UNIVERSITY OF BIRMINGHAM) | 1-5 | |
| | ----- | | TECHNICAL FIELDS SEARCHED (Int. Cl. 3) |
| | | | A 61 K |

The present search report has been drawn up for all claims

| Place of search | Date of completion of the search | Examiner |
|---|---|---|
| THE HAGUE | 11-03-1983 | BRINKMANN C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82